# EUROPEAN PATENT SPECIFICATION

(11) **EP 0 302 381 B1**
(45) Date of publication and mention of the grant of the patent: **27.10.1993**
(21) Application number: 88112138.8
(22) Date of filing: 27.07.1988
(51) Int. Cl.: C12N 15/62

(54) **A fused gene having aequorin gene linked to a functional B-galactosidase gene**
Fusionsgen enthaltend Aequoringen in Verbindung mit einem funktionellen B-galactosidase Gen
Gène fusionné ayant le gène de l'aequorin unie à un gène fonctionnel de B-galactosidase

(30) Priority: 05.08.1987 JP 196031/87
(43) Date of publication of application: 08.02.1989
(73) Proprietor: CHISSO CORPORATION, Osaka (JP)
(72) Inventor: Inouye, Satoshi, Kanazawaku Yokohamashi Kanagawaken (JP); Sakaki, Yoshiyuki, Fukuokashi Fukuokaken (JP)
(74) Representative: Kraus, Walter, Dr.

(56) References cited:
- EP-A- 0 187 519
- EP-A- 0 226 979
- EP-A- 0 245 093
- WO-A-86/01533
- WO-A-86/06742
- Gene, vol. 29, July 1984, pages 27-31, Amsterdam, NL, A. Ullmann "One-step purification of hybrid proteins which have beta-galactosidase activity"
- Biochemistry, vol. 26, no.5, 10th March 1987, pages 1326-1332, Washington DC, US, Prasher et al "Sequence comparisons of complementary DNAs encoding aequorin isotypes"
- Biochemistry, vol. 25, no.26, December 1986, pages 8425-8429, Washington DC, US, S. Inouye et al "Expression of apoaequorin complementary DNA inescherichia coli"

## Description

### BACKGROUND OF THE INVENTION

### 1. Field of the Invention

This invention relates to a fused gene having a β-galactosidase gene linked to the 5' terminus of a gene specifying the biosynthesis of photoprotein aequorin and a process for producing a fused protein using the fused gene.

### 2. Description of the Related Art

Calcium-binding photoprotein aequorin existing in nature is a photoprotein isolated from photogenic jellyfish Aequorea living at the Northern part of Western coast in America.

This protein emits light by oxidation and decomposition of an emitter retained in the protein in the presence of trace calcium (10⁻⁷ M). By utilizing this luminescence, it is possible to measure the concentration of Ca²⁺; hence the aequorin is useful as a reagent or an detecting agent. However, its production quantity is insufficient so that its constant feed cannot be secured.

The present inventors have made extensive research in order to solve the above-mentioned problem. As a result, we obtained cDNA clone of the photoprotein aequorin according to gene engineering method and named the plasmid of the cDNA clone "pAQ440" (see Japanese patent application laid-open No. Sho 61-135,586/1986). Next, by inserting this gene "pAQ440" into a plasmid having an expression promoter inside Escherichia coli, and using the resulting transformed bacterial body, it has become possible to efficiently produce natural type and fused type photogenic protein aequorins inside Escherichia coli (Japanese Patent application Nos. Sho 60-280,259/1985 and Sho 61-248,098/1986).

Further, in order to carry out analyses of the structure and function of the aequorin, we have found various variant aequorin genes according to site-specific mutagenesis and also have established its production process (Japanese patent application Nos. Sho 61-245,108/1986, Sho 61-245,109, Sho 62-126,373/1987 and Sho 62-126,374/1987).

These facts are meaningful in the aspect of protein engineering, and since they lead to elucidations of the mechanism of luminescence of the aequorin and its regeneration mechanism, they have utilization value scientifically and commercially.

In view of the technical situations relative to the aequorin protein, the present inventors have further made research directed to producing a protein having two kinds of functions (activities) by linking a functional protein gene to the 5' terminus of the natural type aequorin gene "pAQ440" i.e. to the N terminus of the formed protein according to recombinant DNA technique and producing a fused protein through the linking inside the bacterial bodies of Escherichia coli. As a result, we have succeeded in obtaining such a protein gene and producing a fused protein by the use of the gene.

The production of this fused protein aims at making it possible to indirectly measuring the function and activity of the protein linked to the above N terminus.

### SUMMARY OF THE INVENTION

As apparent from the foregoing, the object of the present invention is to provide the above-mentioned fused gene and a process for producing a fused protein by the use of the gene.

The present invention in two aspects resides in the following two main constitutions (1) and (2):
(1) A fused gene having a functional β-galactosidase gene linked to the 5' terminus of a gene specifying the biosynthesis of photoprotein aequorin.
(2) A process for producing a fused protein, which comprises using a fused gene obtained by having a functional β-galactosidase gene linked to the 5' terminus of a gene specifying the biosynthesis of photoprotein aequorin.

### BRIEF DESCRIPTION OF THE DRAWINGS

Fig. 1 shows a flowsheet illustrating the construction of an aequorin gene having β-galactosidase gene as its N terminus, relative to Example of the present invention.

Fig. 2 shows an explanatory figure illustrating the base arrangement and the amino acid arrangement at the fused part of β-galactosidase to the aequorin gene, relative to Example of the present invention.

### DETAILED DESCRIPTION OF PREFERRED EMBODIMENTS

The constitution and effectiveness of the present invention will be described below in more detail.

### (1) Preparation of Hin d III fragment containing aequorin gene:

This preparation is carried out in order to make it possible to link a functional protein gene to the N terminus of the aequorin gene.

cDNA clone of photoprotein aequorin (pAQ440) isolated from photogenic Aequorea is first digested with restiction enzymes (e.g. Bcℓ I and Pvu II) to obtain a restriction enzyme fragment containing aequorin cDNA (e.g. Bcℓ I/Pvu II fragment). Such a preparation may be carried out e.g. according to a process of separation and extraction with 3.5% acrylamide gel (electrophoresis method).

### (2) Construction of plasmid piV-18:

The fragment obtained in the above item (1) is subjected to cloning at the cloning site (Bam HI-Hin c II part) of a cloning vector such as cloning vector pUC 18 (Gene, 33, p 103 - 119, 1985, C. Yanisch-Perron et al) to construct plasmid piV-18.

### (3) Construction of pUR-iY:

The piV-18 obtained in the above item (2) is digested with Hin d III, and the resulting fragment is subjected to cloning at the Hin d III part of pUR 228 (EMBO J. vol. 2 (10), p 1791 - 1794, 1983, U. Ruther and B. Muller-Hill) having a protein gene having the function as a cloning vector e.g. β-galactosidase gene (lac Z⁺) and a lac promoter to construct pUR-iY.

Examples of the protein gene having the function of a cloning vector are not only a gene specifying an enzyme activity such as the above-mentioned β-galactosidase gene, but also a gene of protein having DNA-binding activity or a gene specifying the antibody thereof, etc., but the above protein genes are not limited to these.

### (4) Expression in Escherichia coli:

The plasmid pUR-iY obtained in the above item (3) makes it possible to control the expression thereof in Escherichia coli by the use of an inducing agent IPTG (isopropyl-β-thiogalactoside). The steps where a plasmid obtained using other cloning vectors in the above item (3) is used are also almost the same as the above.

### (5) Production in Escherichia coli:

The fused plasmid constructed in the item (3) is subjected to transformation into Escherichia coli e.g. D1210 strain (lac I^{Q}) in a conventional manner to obtain a strain having pUR-iY.

The thus obtained strain is cultivated overnight using a suitable medium e.g. L medium containing 50 µg/mℓ of Ampicillin (bactotryptone (10 g), yeast extract (5 g) and NaCℓ (5 g) each per 1 ℓ). A very small quantity of the resulting culture, e.g. ¹/₁₀₀ thereof is cultivated using the above-mentioned medium (37°C, 3 hours), followed by adding the above-mentioned inducing agent IPTG in a suitable quantity, for example, so as to give a final concentration of 1 mM, and thereafter again cultivating the resulting mixture for 3 hours.

The resulting culture is treated for example as follows:
Bacterial bodies of crude enzyme extract solution are first collected by means of a centrifuge (6,000 g, 10 minutes), followed by adding the bodies to 50 mM of Tris-HCℓ, pH 8.0, (containing 10% glucose), 10 mM of EDTA and lysozyme (1 mg/mℓ), allowing the mixture to stand on ice for one hour, successively subjecting it to centrifugal treatment (12,000 g, 10 minutes) and then obtaining the supernatant.

This supernatant contains the fused protein of the present invention expressed in the above Escherichia coli.

### (6) Measurements of the function of the protein and the activity of the aequorin:

a. The function of the protein obtained in the above item (5) may be measured by reacting the protein with the corresponding reagent. For example, as to the activity of the β-galactosidase in the case of the above item (5), o-nitrophenol formed by subjecting it to enzymolysis using o-nitrophenyl-β-galactoside (hereinafter abbreviated to ONPG; a commercially available product made by Sigma Company) as a substrate is measured with OD₄₂₀. A reaction system consisting of ONPG (10 mg/mℓ) 100 µℓ, enzyme extraction solution 200 µℓ and 100 mµ phosphoric acid buffer 70 µℓ, was reacted at 30°C for 20 minutes and the activity of the resulting protein was measured with OD₄₂₀.
b. Measurement of the aequorin activity:
   The measurement of the aequorin activity may be carried out for example as follows:
   Crude enzyme extraction solution (50 µℓ), a buffer (200 µℓ), Tris-HCl, pH 7.8 (30 mM), EDTA (10 mM), coelenterazine as substrate (5 µℓ, 1 mg/mℓ) and 2-mercaptoethanol (5 µℓ) are mixed, followed by allowing the mixed solution to stand on ice for 2 hours, successively transferring 50 µℓ of the solution into a cuvette of a lumiphotometer (TD-4000, tradename of an instrument made by Laboscience Company), pouring 30 mM of CaCℓ₂ (100 µℓ) therein, measuring the quantity of light emitted and expressing it in terms of relative quantity of light emitted (relative light unit (r.l.u.)) (see Table 1 in Example mentioned later).

In Table 1, D1210 or D1210/pUR288 exhibits only β-galactosidase activity whereas D1210/pUR-iY exhibits both the above activity and the aequorin activity; thus production of a fused protein is shown.

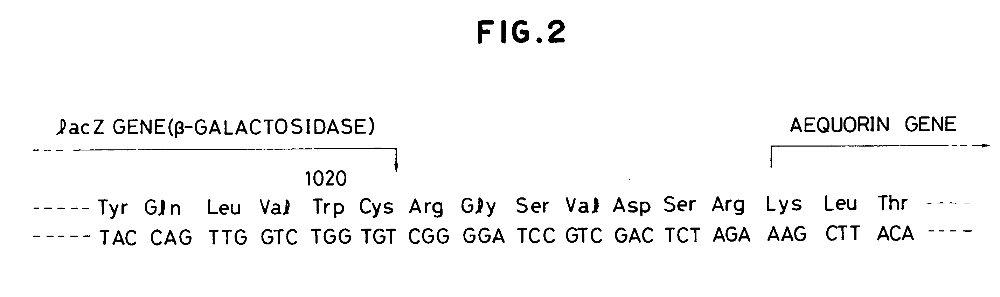

In addition, Fig. 2 shows a base arrangement and an amino acid arrangement at the fused part of β-galactosidase gene and aequorin gene relative to the above pUR-iY.

The present invention will be described by way of Example.

### Example

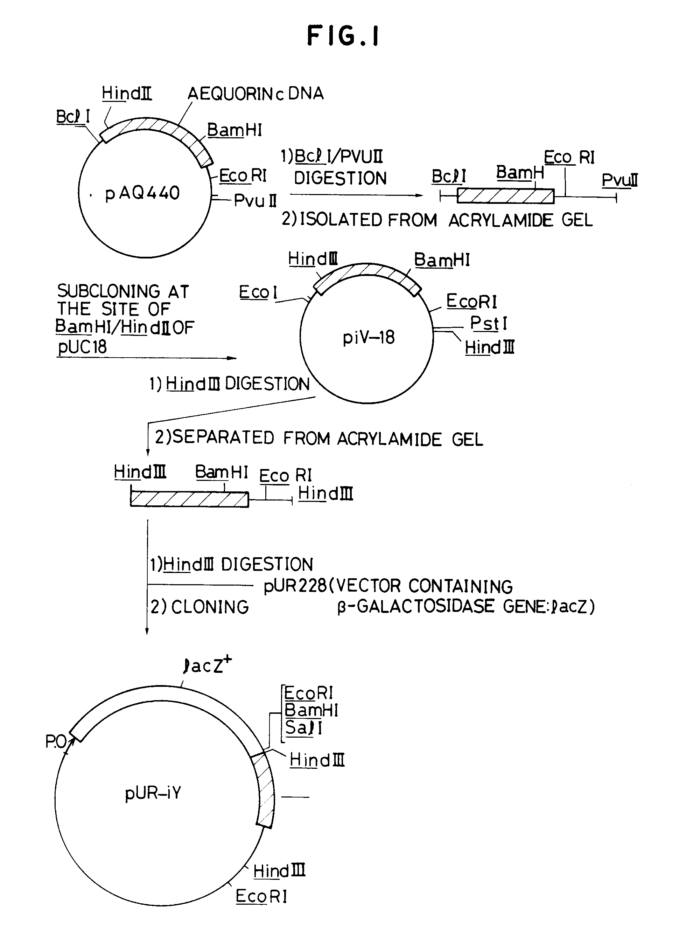

### (1) Fig. 1 shows construction of aequorin protein gene having β-galactosidase gene at the N terminus of the above gene.

From cDNA clone (PAQ440) of photoprotein aequorin isolated from photogenic jellyfish Aequorea is separated and extracted a Bcℓ I/Pvu II fragment containing aequorin cDNA, through the steps of digestion with restriction enzymes Bcℓ I and Pvu II and electrophoresis with 3.5% acrylamide gel. The thus obtained fragment is subjected to cloning at the Bam HI-Hin c II part as the cloning site of a cloning vector pUC 18^{*}¹ (*1: Gene, 33, p 103-119 (985, C. Yanisch - Perron et al)) to construct a plasmid piV-18. Further, after digestion of the piV-18 at Hin d III, a Hin d III fragment containing aequorin gene is similarly separated, followed by subjecting it to cloning at the Hin d III part of pUR228^{*}² vector (*2: EMBO J. Vol. 2 (10), p 1791-1794, 1983, U. Ruther and B. Muller-Hill) having β-galactosidase gene (lac Z⁺) and a lac promoter, to construct pUR-iY. This plasmid pUR-iY has a promoter operator of lac and makes it possible to adjust expression of fused protein in Escherichia coli with an inducing agent IPTG (isopropyl-β-thiogalactoside). The protein produced from the plasmid pUR-iY in Escherichia coli consists of β-galactosidase consisting of 1,021 amino acids, 7 spacer amino acids and 188 amino acids of aequorin protein (apoprotein). Fig. 2 shows the base arrangement and the amino acid arrangement of the fused gene part.

### (2) Production in Escherichia coli:

The fused plasmid pUR-iY constructed in the above item (1) is subjected to transformation into Escherichia coli D1210 strain (lac I^{Q}) in a conventional manner to obtain a strain having pUR-iY.

The resulting strain is cultivated at 37°C overnight in L medium containing 50 µg/mℓ of Ampicillin (bactotryptone (10 g), yeast extract (5 g) and NaCℓ (5 g)), followed by cultivating ¹/₁₀₀ of the quantity of the resulting culture solution at 37°C for 3 hours in the same medium as the above, thereafter adding IPTG so as to give a final concentration of 1 mM, again cultivating the mixture for 3 hours, just thereafter collecting bacterial bodies by means of a centrifuge (6,000 g, 10 minutes), adding to the bacterial bodies the resulting enzyme extraction solution, 50 mM of Tris-HCℓ, pH 8.0 (containing 10% glucose), 10 mM of EDTA and lysozyme (1 mg/mℓ), allowing the mixture to stand on ice for one hour, and thereafter subjecting it to centrifugal separation (12,000 g, 10 minutes) to obtain a supernatant. This supernatant contains a fused protein expressed in Escherichia coli.

### (3) Measurements of β-galactosidase activity and aequorin activity:

a. As to the β-galactosidase activity, o-nitrophenol formed by subjecting it to enzymolysis using o-nitrophenyl-β-galactoside (ONPG, made by Sigma Company) as a substrate was measured with OD₄₂₀. A reaction system consisting of ONPG (10 mg/mℓ) 100 µℓ, enzyme extraction solution (200 µℓ) and 100 mM phosphoric acid buffer (700 µℓ) was reacted at 30°C for 20 minutes and the activity of the resulting protein is measured with OD₄₂₀.
b. Measurement of aequorin activity:
   This measurement is carried out as follows:
   Crude enzyme extraction solution (50 µℓ), a buffer (200 µℓ), Tris-HCl,pH 7.8 (30 mM), EDTA (10 mM), coelenterazine as substrate (5 µℓ, 1 mg/mℓ) and 2-mercaptoethanol (5 µℓ) are mixed, followed by allowing the mixed solution to stand on ice for 2 hours, transferring 50 µℓ of the solution into a cuvette of a lumiphotometer (TD-4000, tradename of an instrument made by Laboscience Company), pouring 30 mM CaCℓ₂ (100 µℓ) therein, measuring the quantity of light emitted and expressing it in terms of relative quantity of light emitted (relative light unit (r.l.u.)). The results are shown in Table 1.

As seen from Table 1, the aequorin activity and the β-galactosidase activity originated from plasmid pUR-iY were detected; hence production of a fused protein wherein β-galactosidase protein was fused with aequorin protein on the side of its N terminus is evident.

**Table 1**

| Production in Escherichia coli | | | | |
|---|---|---|---|---|
| Bacterial body/plasmid | Aequorin activity (rlu/ml) | | β-galactosidase activity ΔOD₄₂₀/20min | |
| | IPTG | | IPTG | |
| | - | + | - | + |
| D1210/- | 0 | 0 | 0.02 | 0.04 |
| D1210/pUR288 | 0 | 0 | 0.02 | 0.30 |
| D1210/pUR-iY | 0.2 | 79.6 | 0.02 | 0.10 |

## Claims

1. A fused gene having a β-galactosidase gene linked to the 5' terminus of a gene specifying the biosynthesis of photoprotein aequorin.

2. A process for producing a fused protein characterized by using a fused gene having a β-galactosidase gene linked to the 5' terminus of a gene specifying the biosynthesis of photoprotein aequorin.

## Patentansprüche

1. Fusioniertes Gen, das ein mit dem 5'-Ende eines die Biosynthese des Photoproteins Aequorin bestimmenden Gens verknüpftes β-Galactosidasegen aufweist.

2. Verfahren zur Herstellung eines fusionierten Proteins, gekennzeichnet durch die Verwendung eines fusionierten Gens, das ein mit dem 5'-Ende eines die Biosynthese des Photoproteins Aequorin bestimmenden Gens verknüpftes β-Galactosidasegen aufweist.

## Revendications

1. Gène fusionné ayant un gène de β-galactosidase relié à l'extrémité 5' d'un gène codant pour la biosynthèse de la photoprotéine aéquonne.

2. Procédé pour produire une protéine fusionnée, caractérisé par l'utilisation d'un gène fusionné ayant un gène de β-galactosidase relié à l'extrémité 5' d'un gène codant pour la biosynthèse de la photoprotéine aéquorine.
